# EUROPEAN PATENT APPLICATION

(11) **EP 4 744 653 A1**
(43) Date of publication of application: **20.05.2026**
(21) Application number: 24213484.9
(22) Date of filing: 18.11.2024
(51) Int. Cl.: A61K 31/12, A61P 1/02, A61P 1/04, A61Q 11/00, A61K 8/35

(54) **PAEONOL AS A SUPPRESSOR OF VOLATILE SULFUR COMPOUND BACTERIAL METABOLITES AND FOR USE IN THE TREATMENT OF DISEASES CAUSED BY BACTERIA PRODUCING VOLATILE SULFOUR COMPOUNDS (VSC) METABOLITES IN THE ORAL CAVITY AND/OR DIGESTIVE SYSTEM**

(71) Applicant: SkyLab AG, 1066 Epalinges (CH)
(72) Inventor: Belous, Elena, 121309 MOSCOW (RU); Yarovaya, Alina Olegovna, 109518 MOSCOW (RU)
(74) Representative: Glawe, Delfs, Moll

(57) **Abstract**

The invention relates to paeonol as a suppressor of volatile sulfur compound bacterial metabolites and the medical and non-medical use of paeonol and paeonol comprising compositions.

## Description

### BACKGROUND OF THE INVENTION

Good oral health is a fundamental prerequisite for overall health and affects the wellbeing and quality of life of every individua. Improper diet, smoking, alcohol intake, and poor oral hygiene practices are the most significant factors influencing the occurrence of various oral diseases. The most effective method for preventing most diseases is a daily oral hygiene by proper mechanical cleaning of the teeth (Tadin et al., 2022).

Neglect of tooth brushing, the use of low-quality cleansing agents or improper brushing technique result in numerous diseases, which deteriorate the life quality and require the doctor's intervention. In particular, such pathologies include dental caries, gingivitis, periodontitis, etc. This is due to the fact that inadequate brushing leads to food particles and bacteria remaining on teeth surface and between the teeth, which can cause the development of the above diseases. As estimated, in WHO European region dental carries affects 50.1% of adult population ("Global oral health status report: towards universal health coverage for oral health by 2030: Summary of the WHO European Region"). Severe dental caries leads to the teeth loss, which has negative impact on human esthetics, functionality, self-esteem, and quality of life (Chan et al., 2021).

Imperfect cleansing is among the causes of inflammation of the dental soft tissues. The inflammation results in destruction of the soft tissue structure, enlargement of periodontal pocket, and bleeding; accordingly, the bacteria can reach the circulation and cause the cardiovascular diseases (Scannapieco, 1999).

Consequently, there is increasing demand for regular dental care to prevent the diseases and subsequent comprehensive treatment. Over the past years the annual growth of the market of dental pastes and tooth brushes exceeds 6% both in Russia, and in the European and American countries. However, according to WHO, almost half of the global population (45% or 3.5 billion people) still suffers from the oral diseases, with three quarters living in low- and middle-income countries. During the last 30 years the incidence of oral-dental diseases increased by one billion cases. This may suggest both inadequate oral hygiene in population, and low efficacy of the used cleaning agents.

The oral flora consists of various microorganisms including viruses, bacteria, fungi, mycoplasma and sometimes protozoa. Bacteria form the predominant component of the oral flora and can amount 300-350 different species. Certain bacterial species are able to adhere to oral mucosa and to the tooth surface leading to biofilm formation. In this biofilm the microorganisms are closely associated and are integrated into the matrix made of bacterial and salivary exopolymers. The bacterial composition in the biofilm undergoes changes with the growth of dental plaque. Many oral pathologies, such as caries, periodontal diseases and peri-implantitis are related to the dental plaque (Vranić et al., 2004). In young people the intensity of dental caries and gingivitis is directly related to the amount and distribution of dental plaque. Most commonly the dental plaque is observed above the gum, in dental neck area, and in fissures. Due to the porous structure of dental plaque the carbohydrates can readily reach the deep layers of plaque. The intake of soft food and considerable amount of readily fermented carbohydrates leads to significant and fast growth of dental plaque. The bacteria entering the composition of dental plaque produce the acids, which destroy the dental enamel causing the defects and holes. In addition, the dental plaque bacteria can cause the gum inflammation (gingivitis) and periodontitis - the periodontal disease, which results in destruction of the tooth supporting tissue (Otten et al., 2012).

Volatile sulfur compound (VSC), such as hydrogen sulfide, methyl mercaptan, dimethyl sulfide, indole etc. are among the metabolites of bacteria inhabiting the dental plaque and subgingival pocket. Dead epithelial cells and glycosylated saliva proteins are the main food sources for oral bacteria. The bacteria producing the volatile sulfur compounds metabolize the protein to produce energy, and this results in degradation of sulfur-containing amino acids and VCS production. Cysteine degradation results in hydrogen sulfide production, while methionine give rise to methyl mercaptan further transforming to dimethyl sulfide (Osama S. et al., 2009). These compounds are known for their very unpleasant odor: hydrogen sulfide smells rotten egg, while methyl mercaptan and dimethyl sulfide are associated with the odor of decaying cabbage. These tree compounds play a key role in development of oral halitosis (Katarzyna H., Marcelina J. et al., 2020).

In the recent years the role of volatile compounds in pathogenesis of periodontal diseases was intensively studied by the researchers. It was found that methyl mercaptan and hydrogen sulfide concentrations in the patients with oral cavity diseases was five-hold compared to the control group (Yeon-Hee Lee et al., 2023). The studies showed that H₂S was higher in the oral cavity air, while higher CH₃SH concentration was observed in periodontal pocket. Hydrogen sulfide (H₂S) is a gas smelling rotten eggs and conventionally considered as toxic. H₂S is a gaseous mediator having numerous functions depending on tissue or organ. The regulation of intracellular oxidation-reduction homeostasis and post-translational protein modification are among the most extensively studied molecular mechanisms of H₂S cell effects. The studies showed that H₂S is directly implicated in onset and development of periodontal diseases: this compound inhibits keratinocyte proliferation in oral cavity, decreases the protein synthesis, and inhibits the collagen synthesis in basal membrane (Greabu et al., 2016).

It was found that apoptotic cell death plays an important role in pathogenesis of various oral cavity diseases. Several studies showed that H₂S is a toxic factor in clinical cases and in animal experiments. The role of hydrogen sulfide premature apoptosis of oral cavity cells. It was shown that high concentrations of physiological H₂S induce apoptosis by various molecular pathways in several cell types including the cells of subgingival pouch. These concentrations induced apoptosis through SOD enzyme inhibition in human gum fibroblasts. This enzyme is essential for ROS production and leads to damage of DNA structure (J. H. Zhang et al., 2010).

Hydrogen sulfide is also involved in production of black stain plaque. This disease is manifested by formation of dark lines or dark dots on the tooth surface. These spots are challenging for the patients, because they could hardly be removed with usual tooth brush or toothpaste and tend to reappear. The researchers showed that black stain plaque is caused by biochemical reactions between the salivary iron and hydrogen sulfide produced by anaerobic bacteria in oral cavity (Celik Z.C).

Contribution to development of physiological and abnormal offensive breath is another clinical effect of H₂S.

Furthermore, hydrogen sulfide molecules produced by *Porphyromonas gingivalis* at certain concentrations have an impact on the development of periodontal inflammation contributing to the synthesis of pro-inflammatory mediators such as IL-8 and IL-6 by epithelial cells. This led to aggravation of local inflammation and excessive neutrophil recruitment (Fox et al., 2012).

It was shown that thiols, such as hydrogen sulfide and methyl mercaptan, are capable of increasing the tissue barrier permeability through unknown mechanism. Most likely, thiols interact with the components of basal membrane and/or intercellular matrix or split the disulfide bonds inducing proteoglycan degradation. Higher permeability leads to entry of adverse and abnormal molecules in the gum tissue thus contributing to development of various periodontal diseases.

In addition, methyl mercaptan impaired proliferation of epithelial cells in gum. The studies of the effects of volatile thiol compounds on protein metabolism in human gum fibroblasts showed that methyl mercaptan reduced total protein synthesis by 35%. This, in turn, was associated with decrease in amount of collagenase-digestible protein. Therefore, the cells exposed to CH₃SH exhibited low collagen level and fast collagen degradation (Milella, 2015).

However, sulfur-containing compounds may appear in the oral cavity not only due to proliferation of pathogenic bacteria, but also due to gastrointestinal diseases. The factors responsible for the appearance of sulfur-containing compounds may include alcohol consumption, smoking, dietary habits, diabetes mellitus, and obesity. Furthermore, elevation of thiol compound level in the oral cavity may be caused by stress and vigorous physical activity (Hampelska et al., 2020).

Cleansing components use in the up-to-date toothpastes are mainly directed on effective removal of dental plaque, destruction of biofilms and control of pathogenic bacteria capable to cause dental lesions. In addition, taking into consideration modern trends towards environmental-friendly products, in many compositions plant-derived compounds, proteases, and essential oils known for their antibacterial properties are used. Many of them can exhibit anti-inflammatory and analgesic effects allowing to alleviate and eliminate the sequalae of periodontal diseases. In order to control halitosis, the flavoring agents capable to mask specific odor of sulfur compounds are added to the toothpastes.

Statistics shows that despite broad variety of available dental products the challenge of poor cleansing is still actual globally. As is evident from the study, available components use antibacterial and antiseptic activities for liquidation of dental plaque. However, the manufacturers of dental care products do not consider sulfur-containing molecules as toxic and pathogenic, and as potential causative agents of periodontal diseases. The strategy of removal of bacterial strains capable of producing such molecules can be justified only partially, because the concentration of such molecules in the oral cavity depends also upon condition of human gastrointestinal system and other factors rather than just on the presence of pathogenic bacteria. For this reason alternative approaches to search and selection of cleansing agents and agents preventing the dental plaque emergence are clearly needed.

In the present invention aimed at the control of oral cavity contamination not only by removal of dental plaque but also by regulation of gaseous compound level thus impacting black stain plaque, an active component paeonol is used.

Paeonol or 4-methoxy-2-hydroxyacetophenone is an organic phenolic compound found in peony, such as *Paeonia suffruticosa,* in *Arisaema erubescens* or in *Dioscorea japonica.* Peony has also a long history of clinical use in China. The roots of the plant due to their analgesic and antiviral properties have been traditionally used as a remedy for toothache, abdominal pain, rubella, measles, and malaria (Bae et al., 2023).

*In vitro* and in the animal models a number of biological and pharmacological paeonol effects have been observed, for instance, inhibition of collagen-induced platelet aggregation and alleviation of inflammatory response in respiratory tract, coronary arteries, macrophages, and microglia cells. Accumulated evidence suggests that paeonol can be a promising neuroprotective or anti-neurodegenerative agent due to anti-inflammatory properties and capability of free radical quenching; paeonol protects the neurons against damage caused by oxygen-glucose deficit and against neurotoxicity (Lin et al., 2015). Topical paeonol products can be used for various dermal diseases, such as eczema, dermatitis, itching, mosquito and bug bites, and also have some beneficial effects against allergic rhinitis and as a cold prevention agent.

Paeonol was reported as the inhibitor of free radical formation and platelet aggregation. As was demonstrated in the animal model (a rat paw) of inflammatory pain, carrageenan-evoked thermal hyperalgesia, paeonol is a potent therapeutic agent capable of inhibiting the production of pro-inflammatory cytokines and stimulating the anti-inflammatory molecule production (Chou, 2003).

Due to anti-inflammatory properties paeonol is widely used for treatment of various dermal diseases. In addition, it was shown that the use of paeonol down-regulates the production of matrix metalloproteinase 1, which is responsible for skin inflammation caused by UV irradiation(L. Zhang et al., 2019).

Therefore, numerous prior studies disclosed paeonol as neuroprotective, anti-inflammatory, and anticancer agent capable of alleviating severe inflammatory pain and protect the skin cells. A number of unique properties were demonstrated, which make paeonol potentially beneficial for medical use. However, the authors of the present invention unexpectedly found that particular paeonol concentrations contribute to effective cleansing of oral cavity due to reduction of gaseous substances levels.

Paeonol is implemented in several patent publications and commercial products.

From the prior art a toothpaste Ezeno Organic Toothpaste is known containing ginseng and tea tree and based on the certified organic oils. The other ingredients of said toothpaste include paeonol from peony extract, glycosides, and paeoniflorin. The manufactures recommend to use this product for general oral hygiene, prevention of inflammation and to control the bacterial pathogens. Furthermore, the authors do not consider the use of paeonol as a cleansing component of a composition, it is described as an anti-inflammatory and antiseptic agent.

The toothpaste Orecare U Smile is also known; this paste is enriched with the plant extracts and is used to alleviate stomatitis, gum edema and hemorrhage, halitosis. Paeonol being a principal active ingredient of this product is used to maintain the gum health and to reduce the dental plaque. Though paeonol serves as a cleansing agent in this toothpaste, the authors suggest reduction of dental plaque as a cleansing mechanism, and do not consider the mechanisms disclosed in the present invention.

The composition provided in patent publication CN108030706A of 15.12.2017 comprises paeonol and was developed for oral care. The composition can be used as a toothpaste and comprises a strontium salt as a principal component in addition to paeonol. However, the authors consider the use of paeonol as an antiallergic agent, and said use is not related to the application field of the present invention.

Another known toothpaste (see patent publication CN102895147 of 25.09.2012), comprises paeonol, sesamin, eugenol and dipotassium glycyrrhizinate. Said invention disclosing paeonol as anti-inflammatory and hemostatic agent is directed at alleviation of pain and inflammation in humans suffering from periodontitis and gingivitis. It is proposed that such composition is not allergenic and can be used to alleviate the allergy symptoms. Thus, the field of application of the disclosed composition is not related to the field of the present invention.

### EMBODIMENTS OF THE INVENTION

The authors unexpectedly found that the use of paeonol is capable to reduce the gaseous substance in the oral cavity, due to this fact the composition comprising said component can provide effective cleansing by suppression of negative effects caused by high concentrations of thiol compounds rather than just through removal of dental plaque.

Additional advantages and effects obvious to the persons skilled in the art are disclosed throughout the document or directly follow from specification of the invention.

### EXAMPLES

Example 1. Assessment of the effect of various paeonol concentrations on hydrogen sulfide and methyl mercaptan levels over 6- and 12-hour observation periods.

For the study the biomaterial was collected. Saliva specimens were collected from 4-6 healthy individuals, which were neither smokers nor received the medicaments. The specimens were collected 1.5-2 hours after the last food/liquid intake. The individuals chewed the piece of parafilm 5x3 to 5x5 cm to stimulate saliva production. Saliva was collected into clean 20 ml vials with the use of glass funnels. The vials and funnels after the use were washed with the detergent, rinsed with distilled water and kept at 121 °C overnight. The collected saliva specimens were averaged by mixing of the obtained specimen with the use of magnetic stirrer and used immediately.

For the solution preparation methanol (chemically pure grade, Chimmed) or deionized water (Merck Millipore) were used; paeonol of 99% purity was used as a test component. 2% zinc gluconate solution known for its deodorizing properties was used as a positive control (M. Abendrot et al. 2018, J. Schwartz et al. 2006). The solutions of all components were prepared in 1ml of solvent on every occasion immediately before the analysis.

Despite known deodorizing activity of zinc gluconate, the use of this compound in the formulations of toothpastes and other hygienic compositions is hampered because of instability on heating. In the course of heating zinc ions can form insoluble Zn(OH)₂ complex and precipitate. Furthermore, at pH 7-8 zinc gluconate forms insoluble complexes with OH⁻, and this can also result in precipitation, which is of particular concern in the event of liquid formulations for oral care, such as rinses, solutions for irrigator, foams, etc. (Yajun Zheng et al, 2023).

20ml of deionized water was added to 0.6 g of dry nutritive formula to obtain nutritive medium. After mixing the medium was boiled for 1 min and then poured into vials, sealed with screw closures equipped with silicone-Teflon septa and sterilized at 121 °C for 15 min.

2 ml of the averaged saliva mixture, 0.85 ml of deionized water, 0.15 ml of thioglycolate medium, and 40 mm³ of the component solution at prespecified concentration were added sequentially into 20 ml vial to obtain the working mixture. The vial containing the working mixture was sealed with screw closures equipped with silicone-Teflon septum and shaken for 1 min for better mixing. The series of 4-5 samples was prepared including 3 samples with added 40 mm³ of the component solutions at different concentrations, 1 sample with added 40 mm³ of deionized water (negative control) and, if necessary, 1 sample with added 40 mm³ of methanol. Preparation and analysis of the sample series for each component was made in triplicate for 3 parallel measurements.

For the analysis the prepared sample series was incubated in vertical position in the autosampler thermostat without shaking at 37 °C for 6 h. Autosampler was used for vapor sampling from the headspace and injection into vaporizer of chromatograph. Incubation and analysis cycle was repeated once again. In such a manner the data were obtained at 6 h and 12 h after mixing and the mixture placement into the thermostat.

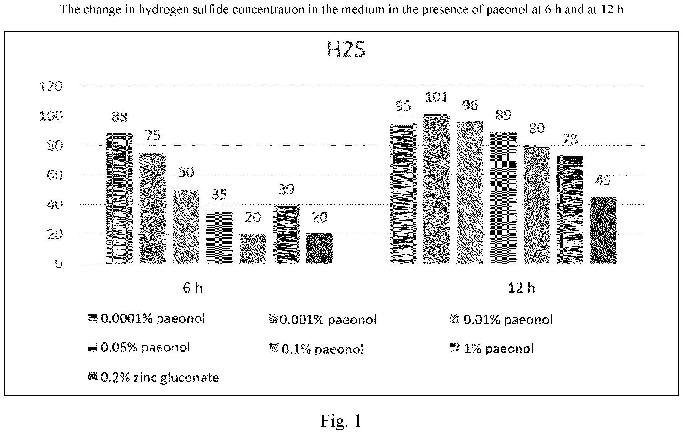

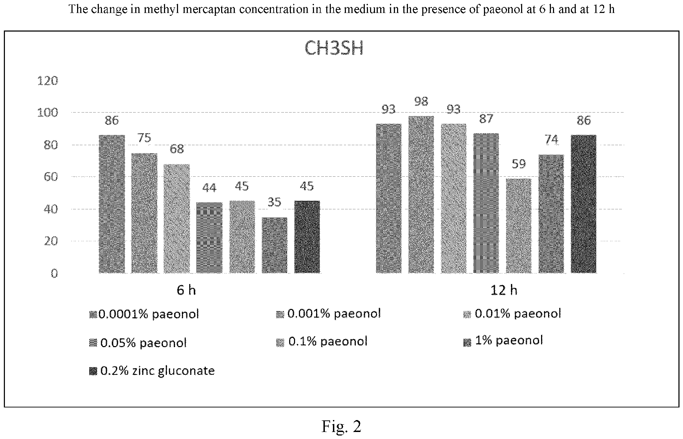

The chromatograms were recorded and processed with the use of GCMS Solution 2.72 package. The peaks identification was based on retention time and ion ratio. The retention time of hydrogen sulfide was 3.34 min, with ion ratio 34: 33: 36 as 100%: 36%: 4.3%. the retention time of methyl mercaptan was 6.91 min, with ion ratio 47: 45: 48, as 100%: 52%: 87%. As a result of analysis a chromatographic peak area was assessed by ion 34 for hydrogen sulfide and by ion 47 for methyl mercaptan. The changes in gaseous substance concentration over the time are shown in Figures 1 and 2 and in Table 1.

**Table 1. The measurements of methyl mercaptan and hydrogen sulfide levels in the medium at 6 h and 12 h time points**

| Time points | Gaseous substance | 1% paeonol | 0.1% paeonol | 0.05% paeonol | 0.01% paeonol | 0,001% paeonol | 0,0001 % paeonol | 0.2% zinc gluconate |
|---|---|---|---|---|---|---|---|---|
| | | | Concentration of gaseous substances, % | | | | | |
| 6 h | Hydrogen sulfide | 39.0 | 20.5 | 35.4 | 49.8 | 75.4 | 88.0 | 20.2 |
| 12 h | Hydrogen sulfide | 73.0 | 80.4 | 89.0 | 96.0 | 101.4 | 94.5 | 45.2 |
| 6 h | Methyl mercaptan | 34.6 | 21.7 | 43.8 | 68.3 | 69.7 | 85.8 | 45.2 |
| 12 h | Methyl mercaptan | 73.6 | 58.6 | 86.6 | 93.5 | 98.2 | 92.6 | 85.7 |

As is evident in Figures 1 and 2 and also from table 2, 0,0001-1% paeonol solutions are capable of reducing the gaseous substance levels. At the timepoint of 6 hours six different paeonol concentrations were tested, and with 0.1% paeonol solution maximal reduction of hydrogen sulfide and methyl mercaptan levels was achieved and increasing the concentration of paeonol to 1% does not contribute significantly to the reduction of the above sulfur-containing volatile substances. When considering the timepoint of 12 hours, it can also be concluded that the effectiveness in reducing the concentration of methyl mercaptan and hydrogen sulfide at 0.1% and 1% concentrations of paeonol is comparatively the same. Lowering of active components concentrations is associated with weaker effect on sulfur compound levels. 0.1% paeonol solution reduced hydrogen sulfide production at 6 hours as effectively as 0.2% zinc gluconate solution, but at 12 hours this trend is not maintained, and paeonol demonstrates lower efficacy compared to zinc. However, in the tests with methyl mercaptan 0.1% paeonol solution shows much higher efficacy against production of said gaseous substance both at 6h and at 12h timepoints.

Thus, the authors demonstrated that various concentrations of paeonol solution can control the levels of sulfur compounds in oral cavity, furthermore, the discovered effect maintained for a long period of time (for 6 hours), then weakened to some extent but was still evident at the later timepoint (12 hours).

### REFERENCES

1. Bae, H. J., Kim, J. Y, Choi, S. H., Kim, S. Y, Kim, H. J., Cho, Y E., Choi, Y Y, An, J. Y, Cho, S. Y, Ryu, J. H., & Park, S. J. (2023). Paeonol, the active component of Cynanchum paniculatum, ameliorated schizophrenia-like behaviors by regulating the PI3K-Akt-GSK3β-NF-κB signalling pathway in MK-801-treated mice. Journal of Ethnopharmacology, 314. https://doi.org/10.1016/j.jep.2023.116627
2. Chou, T. C. (2003). Anti-inflammatory and analgesic effects of paeonol in carrageenan-evoked thermal hyperalgesia. British Journal of Pharmacology, 139(6). https://doi.org/10.1038/sj.bjp.0705360
3. Fox, B., Schantz, J. T., Haigh, R., Wood, M. E., Moore, P. K., Viner, N., Spencer, J. P. E., Winyard, P. G., & Whiteman, M. (2012). Inducible hydrogen sulfide synthesis in chondrocytes and mesenchymal progenitor cells: Is H2S a novel cytoprotective mediator in the inflamed joint? Journal of Cellular and Molecular Medicine, 16(4). https://doi.org/10.1111/j.1582-4934.2011.01357.x
4. Greabu, M., Totan, A., Miricescu, D., Radulescu, R., Virlan, J., & Calenic, B. (2016). Hydrogen sulfide, oxidative stress and periodontal diseases: A concise review. In Antioxidants (Vol. 5, Issue 1). https://doi.org/10.3390/antiox5010003
5. Hampelska, K., Jaworska, M. M., Babalska, Z. L., & Karpiński, T. M. (2020). The role of oral microbiota in intra-oral halitosis. In Journal of Clinical Medicine (Vol. 9, Issue 8). https://doi.org/10.3390/jcm9082484
6. Lin, C., Lin, H. Y, Chen, J. H., Tseng, W. P., Ko, P. Y, Liu, Y S., Yeh, W. L., & Lu, D. Y. (2015). effects of paeonol on anti-neuroinflammatory responses in microglial cells. International Journal of Molecular Sciences, 16(4). https://doi.org/10.3390/ijms16048844
7. Milella, L. (2015). The negative effects of volatile sulphur compounds. Journal of Veterinary Dentistry, 32(2). https://doi.org/10.1177/089875641503200203
8. Otten, M. P. T., Busscher, H. J., Abbas, F., van derMei, H. C., & van Hoogmoed, C. G. (2012). Plaque-left-behind after brushing: Intra-oral reservoir for antibacterial toothpaste ingredients. Clinical Oral Investigations, 16(5). https://doi.org/10.1007/s00784-011-0648-2
9. Hampelska K, Jaworska MM, Babalska ZL, Karpiński TM. The Role of Oral Microbiota in Intra-Oral Halitosis. J Clin Med. 2020 Aug 2;9(8):2484. doi: 10.3390/jcm9082484. PMID: 32748883; PMCID: PMC7465478.
10. Scannapieco, F. A. (1999). Role of Oral Bacteria in Respiratory Infection. Journal of Periodontology, 70(7). https://doi.org/10.1902/jop.1999.70.7.793
11. Tadin, A., Guberina, R. P., Domazet, J., & Gavic, L. (2022). Oral Hygiene Practices and Oral Health Knowledge among Students in Split, Croatia. Healthcare (Switzerland), 10(2). https://doi.org/10.3390/healthcare10020406
12. Vranić, E., Lacević, A., Mehmedagić, A., & Uzunović, A. (2004). Formulation ingredients for toothpastes and mouthwashes. In Bosnian journal of basic medical sciences / Udruzenje basicnih mediciniskih znanosti = Association of Basic Medical Sciences (Vol. 4, Issue 4). https://doi.org/10.17305/bjbms.2004.3362
13. Zhang, J. H., Dong, Z., & Chu, L. (2010). Hydrogen sulfide induces apoptosis in human periodontium cells. Journal of Periodontal Research, 45(1). https://doi.org/10.1111/j.1600-0765.2009.01202.x
14. Zhang, L., Li, D. chang, & Liu, L. fang. (2019). Paeonol: pharmacological effects and mechanisms of action. In International Immunopharmacology (Vol. 72). https://doi.org/10.1016/j .intimp.2019.04.033
15. M. Abendrot, U. Kalinowska-Lis. (2018). Zinc-containing compounds for personal care applications. In International Journal of Cosmetic Science (Vol. 40). https://doi.org/10.1111/ics. 12463
16. James R. Schwartz, Randall G. Marsh, Zoe Diana Draelos. (2006). Zinc and Skin Health: Overview of Physiology and Pharmacology. In Dermatologic Surgery. https://doi.org/10.1111/j.1524-4725.2005.31729
17. Osama S. Duwaji B.A. (2009). The bacterial metabolic processes that produce volatile sulfur compounds in the oral cavity. In Boston University.
18. Lee, YH., Shin, SI. & Hong, JY (2023). Investigation of volatile sulfur compound level and halitosis in patients with gingivitis and periodontitis. In Sci Rep 13, 13175. https://doi.org/10.1038/s41598-023-40391-3
19. Celik ZC, Cakiris A, Yanikoglu F, Abaci N, Ekmekci SS, Ilgin C, et al.. Metagenomic analysis of black-stained plaques in permanent dentition. Arch Oral Biol. (2021) 128:105171. 10.1016/j.archoralbio.2021.105171.
20. Yajun Zheng, Min Guo, Chaoxia Cheng, Junru Li, Yuanjing Li, Zhixuan Hou, Ying Ai (2023). Structural and physicochemical characteristics, stability, toxicity and antioxidant activity of peptide-zinc chelate from coconut cake globulin hydrolysates. LWT, https://doi.org/10.1016/j.lwt.2022.114367.

## Claims

1. Use of paeonol as a suppressor of volatile sulfur compound (VSC) bacterial metabolites, said VSC metabolites comprising hydrogen sulfide, methyl mercaptan and dimethyl sulfide.

2. *Ex-vivo* method of suppressing VSC bacterial metabolites, said method comprising the step of treating a composition comprising VSC bacterial metabolites with paeonol or a composition comprising paeonol, and said VSC metabolites comprise hydrogen sulfide, methyl mercaptan and dimethyl sulfide.

3. The *ex-vivo* method of claim 2, wherein said composition comprises paeonol as per the following wt% ranges in said composition: 0.00010 - 1.0000, 0.0005 - 0.9000, 0.001 - 0.800, 0.005 - 0.600, 0.007 - 0.500, 0.010 - 0.400, 0.010 - 0.300, 0.010 - 0.200, 0.010 - 0.100, 0.005 - 1.000, 0.006 - 0.900, 0.007 - 0.800, 0.008 - 0.600, 0.009- 0.500, 0.010 - 0.400, 0.010 - 0.300, 0.010 - 0.200, 0.010 - 0.100, or any combination of said upper with said lower ranges endpoints.

4. Paeonol or composition comprising paeonol for use in the prophylaxis or treatment of a disease or a symptom of a disease in a subject, wherein said disease is selected from at least one of the following: intraoral halitosis, Priestley plaque, dyspepsia, indigestion, regurgitation, *Helicobacter pylori* infection, gastric lesion and a esophago-gastric mucosa disease.

5. Paeonol or composition comprising paeonol for use in the prophylaxis or treatment of a disease or a symptom of a disease in a subject, wherein said disease is caused by bacteria producing VSC metabolites in the oral cavity and/or digestive system of said subject, and said VSC metabolites comprise hydrogen sulfide, methyl mercaptan and dimethyl sulfide.

6. The paeonol or the composition comprising paeonol for use of claim 4 or claim 5, wherein said subject is of risk of a hydrogen sulfide intoxication.

7. The composition comprising paeonol for use of any of claims 4-6, wherein said composition is an oral care composition.

8. The composition comprising paeonol for use of any of claims 4-7, wherein said composition
i) is a formulation selected from the following: film, aerosol, suspension, solution, tincture, cream, paste, lotion, ointment, gel, powder, granulate;
and/or
ii) is selected from the following: toothpaste; tooth powder; tooth gel; oral foam; oral gel; chewing gum; sweet; candy; mouth rinse; coating of said composition on dental floss or toothbrush.

9. The composition comprising paeonol for use of any of claims 4-8, wherein said composition comprises paeonol as per the following wt% ranges in said composition: 0.00010 - 1.0000, 0.0005 - 0.9000, 0.001 - 0.800, 0.005 - 0.600, 0.007 - 0.500, 0.010 - 0.400, 0.010 - 0.300, 0.010 - 0.200, 0.010 - 0.100, 0.005 - 1.000, 0.006 - 0.900, 0.007 - 0.800, 0.008 - 0.600, 0.009- 0.500, 0.010 - 0.400, 0.010 - 0.300, 0.010 - 0.200, 0.010 - 0.100 or any combination of said upper with said lower ranges endpoints.

10. Non-medical use of paeonol or non-medical use of a composition comprising paeonol, wherein said non-medical use comprises the suppression of VSC metabolites, preferably in the oral cavity of a subject, and wherein said VSC metabolites comprise hydrogen sulfide, methyl mercaptan and dimethyl sulfide.

11. The non-medical use of paeonol or the non-medical use of a composition comprising paeonol of claim 10, wherein said subject is of risk of a hydrogen sulfide intoxication.

12. The non-medical use of the composition comprising paeonol of claim 10 or claim 11, wherein said composition is an oral care composition.

13. The non-medical use of the composition comprising paeonol of any of claims 10-12, wherein said composition
i) is a formulation selected from the following: film, aerosol, suspension, solution, tincture, cream, paste, lotion, ointment, gel, powder, granulate;
and/or
ii) is selected from the following: toothpaste; tooth powder; tooth gel; oral foam; oral gel; chewing gum; sweet; candy; mouth rinse; coating of said composition on dental floss or toothbrush.

14. The non-medical use of the composition comprising paeonol of any of claims 10-13, said composition comprises paeonol as per the following wt% ranges in said composition: 0.00010 - 1.0000, 0.0005 - 0.9000, 0.001 - 0.800, 0.005 - 0.600, 0.007 - 0.500, 0.010 - 0.400, 0.010 - 0.300, 0.010 - 0.200, 0.010 - 0.100, 0.005 - 1.000, 0.006 - 0.900, 0.007 - 0.800, 0.008 - 0.600, 0.009- 0.500, 0.010 - 0.400, 0.010 - 0.300, 0.010 - 0.200, 0.010 - 0.100 or any combination of said upper with said lower ranges endpoints.

15. The use of claim 1, the *ex-vivo* method of claim 2 or claim 3, the paeonol or the composition comprising paeonol for use of any of claim 4-6, the composition comprising paeonol for use of any of claim 7-9, the non-medical use of paeonol or composition comprising paeonol of claim 10 or claim 11, the non-medical use of the composition comprising paeonol of any of claims 12-14, wherein said paeonol has the CAS number 552-41-0;
and/or
the *ex-vivo* method of claim 2 or claim 3, the composition comprising paeonol for use of any of claim 4-9, the non-medical use of the composition comprising paeonol of any of claims 10-14, wherein composition does not comprise are least one of the following components:
i) zinc gluconate;
ii) strontium or a salt thereof;
iii) sesamine and/or eugenol and/or glycyrrhizinate dicalium.
